# EUROPÄISCHE PATENTSCHRIFT

(11) **EP 1 274 745 B1**
(45) Veröffentlichungstag und Bekanntmachung des Hinweises auf die Patenterteilung: **17.01.2007**
(21) Anmeldenummer: 01915369.1
(22) Anmeldetag: 16.03.2001
(51) Int. Cl.: C08F 8/42, H01F 1/44, G01N 33/543

(54) **MAGNETISCHE, SILANISIERTE TRÄGERMATERIALIEN AUF BASIS VON POLYVINYLALKOHOL**
MAGNETIC, SILANISED POLYVINYLALCOHOL-BASEDCARRIER MATERIALS
MATERIAUX SUPPORTS MAGNETIQUES ET SILANISES, A BASE D'ALCOOL POLYVINYLIQUE

(30) Priorität: 22.03.2000 DE 10013995
(43) Veröffentlichungstag der Anmeldung: 15.01.2003
(73) Patentinhaber: chemagen Biopolymer-Technologie AG, 52499 Baesweiler (DE)
(72) Erfinder: PARKER, W., Jeffrey, Halifax, NS, B3K5Y1 (CA); OSTER, Jürgen, 52134 Herzogenrath (DE); A BRASSARD, Lothar, 52525 Heinsberg (DE)
(74) Vertreter: Wolff, Felix
(86) Internationale Anmeldenummer: PCT/EP2001/003061
(87) Internationale Veröffentlichungsnummer: WO 2001/070831

(56) Entgegenhaltungen:
- EP-A- 0 235 526
- WO-A-99/17869
- DE-A- 4 018 523
- DE-A- 19 528 029
- GB-A- 1 556 584
- GB-A- 2 267 283
- US-A- 4 818 624

## Beschreibung

Gegenstand der vorliegenden Erfindung sind magnetische polymere Trägermaterialien auf Basis von Polyvinylalkohol, deren Oberfläche zumindest teilweise silanisiert ist, Verfahren zur Silanisierung der Oberfläche solcher Materialien und die Verwendung der magnetischen, silanisierten Trägermaterialien zur Isolierung von biologischem Material, vorzugsweise zur Isolierung von Nucleinsäuren.

Magnetische, polymere Trägermaterialien, insbesondere Polymerteilchen, werden im zunehmenden Maße in der Biochemie und der medizinischen Diagnostik zur Abtrennung von Zellen, Proteinen und Nucleinsäuren verwendet. Der Einsatz von magnetischen Trägermaterialien bietet gegenüber herkömmlichen Separationsmethoden den Vorteil, daß die beladenen Trägermaterialien mit Hilfe magnetischer Kräfte einfach und rasch von den übrigen Bestandteilen einer Probe abgetrennt werden können. Magnetische perl- bzw. kugelförmige Polymerpartikel auf Basis von Polyvinylalkohol mit einer engen Korngrößenverteilung in einem Bereich unter 10 µm haben sich für solche Trennverfahren als besonders geeignet herausgestellt (WO 97/04862).

Es ist weiterhin bekannt, daß bestimmte biologische Materialien, insbesondere Nucleinsäuren, aus ihrer natürlichen Umgebung nur unter erhöhtem Aufwand isoliert werden können. Dies liegt oft daran, daß entsprechende biologische Proben meist weitere, feste und/oder gelöste Verbindungen, wie Proteine, enthalten, die die Isolierung beeinträchtigen können bzw. daran, daß die Nucleinsäuren sehr oft nur in geringen Konzentrationen in der zu untersuchenden biologischen Probe vorhanden sind.

Um dennoch die Vorteile einer Isolierung von Nucleinsäuren aus biologischen Proben unter Einsatz magnetischer Partikel nutzen zu können, wurde u. a. vorgeschlagen, Nucleinsäuren mit Hilfe von magnetischen Partikel mit einer Glasoberfläche, die im wesentlichen porenfrei ist, zu isolieren (WO 96/41811). Diese Teilchen müssen eine bestimmte Zusammensetzung, d. h. ihre Glasoberfläche muß eine bestimmte Zusammensetzung aufweisen, um die gewünschte Wirksamkeit zu erzielen, außerdem ist ein relativ aufwendiger Prozeß zum Herstellen dieser Teilchen notwendig, um die notwendige Sinterung der Glasoberfläche zu erreichen.

Aufgabe der vorliegenden Erfindung war es daher, weitere magnetische Trägermaterialien zur Isolierung und/oder Reinigung von biologischen Materialien, vorzugsweise Nucleinsäuren aus biologischen Proben zur Verfügung zu stellen, die vorzugsweise für Nucleinsäuren hoch spezifisch sind und damit auch automatische Diagnostikverfahren erlauben. Weiterhin sollen die Trägermaterialien unkompliziert und kostengünstig hergestellt werden können.

Erfindungsgemäß wird dies durch Bereitstellung von magnetischen Trägermaterialien auf Basis von Polyvinylalkohol erreicht, deren Oberfläche zumindest teilweise silanisiert und ggf. mit Biomolekülen koppelnden Affinitätsliganden ausgerüstet ist.

Diese magnetischen Trägermaterialien können als Filter oder Membran oder als Partikel gestaltet sein. Vorzugsweise liegt das magnetische Trägermaterial als perl- oder kugelförmige Partikel vor, wobei diese Partikel vorzugsweise eine Partikelgröße von 0,2 bis 50 µm, besonders bevorzugt 0,5 bis 5 µm, aufweisen. Neben der vorzugsweise perl- und kugelförmigen Gestalt der Partikel soll deren Teilchengrößenverteilung in einem möglichst engen Bereich liegen.

Verfahren zur Herstellung von magnetischen Polyvinylalkoholträgermaterialien, vorzugsweise in perlförmiger Partikelgestaltung, sind aus der DE-41 27 657 und aus der WO 97/04862 bekannt, deren Offenbarung bezüglich der Herstellungsverfahren von Trägermaterialien hiermit als Referenz eingeführt wird. Gemäß den bekannten Verfahren lassen sich magnetische Partikel mit einer sehr engen Partikelgrößenverteilung und mit Partikelgrößen von 1 bis 4 µm, wie sie insbesondere zur Isolierung von Biosubstanzen in Suspension sowie für diagnostische Medizin verwendet werden, herstellen.

Dabei werden die Polyvinylalkohol-Partikel durch Zugabe von bestimmten EmulgatorMischungen zu der Ölphase der Wasser in Öl Emulsion hergestellt. Als Emulgatoren, die als Zusätze zu der Ölphase gegeben werden, eignen sich Propylenoxid-Äthylenoxid-Blockcopolymere, Sorbitan-Fettsäureester, Komplexmischester aus Pentaerythrit-Fettsäureester mit Citronensäure, Polyäthylenglykol-Castoröl-Derivate, Blockcopolymere aus Rizinusöl-Derivaten, Polyäthylenglykole, modifizierte Polyester, Polyoxyäthylen-Sorbitan-Fettsäureester, Polyoxyäthylen-Polyoxypropylen-Äthylendiamin-Blockcopolymere, Polyglyceryl-Derivate, Polyoxyäthylen-Alkohol -Derivate, Alkylphenylpolyäthylenglykol-Derivate, Polyhydroxyfettsäure-Polyäthylenglykol-Blockcopolymere, Polyäthylenglykol-Ätherderivate. Substanzen dieser Art sind im Handel u.a. unter der Handelsbezeichnung: Pluronic®, Synperonic®, Tetronic®, Triton®, Arlacel®, Span®, Tween®, BrijOR, ReneXOR, Hypermer®, Lameform®, Dehymuls® oder Eumulgin® bekannt.

Um einheitliche, perlförmige Polymer-Partikel vorzugsweise mit Partikelgrößen von 0,5-10 µm zu erhalten, wird in die Ölphase eine Mischung aus mindestens zwei, vorzugsweise drei bis vier der genannten oberflächenaktiven Substanzen zugegeben. Vorzugsweise wird eine lipohile Emulgatorkomponente mit mindestens einem Emulgator gemischt, der semi-hydrophile Eigenschaften aufweist, d.h. der sowohl wasser- als auch öllöslich ist. Emulgatoren, die die letzteren Eigenschaften erfüllen sind z.B.: Äthylenoxid-Propylenoxid-Blockcopolymer-Derivate mit überwiegendem Äthylenoxid-Anteil, Polyäthylenglykolhexadecyläther, kürzerkettige Polyoxyäthylen- Sorbitan-Fettsäureester, Polyäthylenglykole oder kürzerkettige Sorbitan-Fettsäureester. Die Konzentration der Emulgatoren in der Ölphase beträgt in der Regel 2-6 Vol.%, vorzugsweise 3.5-5.0 Vol-%. In Bezug auf Feinheit und enge Partikelgrößenverteilung der Polymertröpfchen sind solche Emulgatormischungen von Vorteil, die mindestens zwei lipophile Komponenten und einen semi-hydrophilen Emulgator enthalten. Die Konzentration des semi-hydrophilen Emulgators liegt in der in der Regel zwischen 15 und 30 Vol%, bezogen auf die Gesamtemulgatormenge. Neben der Feinheit der Partikel zeigen die Partikel eine perlförmige Gestalt.

Neben den Emulgatoren für die Ölphase tragen auch spezielle oberflächenaktive Substanzen, die in der wäßrigen Polymerphase löslich sind, zur Verbesserung der Emulsionssqualität vor allem von Polyvinylalkohollösungen mit niedrigen Molekulargewicht (Mowiol, Clariant GmbH, Frankfurt am Main, BRD) bei. Darüber hinaus gelingt es, die in fester Form zugesetzten magnetischen Kolloide durch Zugabe ionischer Emulgatoren fein zu dispergieren. Beispiele für solche Emulgatoren, die auch als binäre Mischungen eingesetzt werden können, sind: Serum Albumin, Gelatine, aliphatische und aromatische Sulfonsäure-Derivate, Polyäthylenglykole, Poly-N-Vinylpyrrolidon oder Celluloseacetat-butyrat. Die Mengen der eingesetzten Emulgatoren betragen in der Regel 0.01 - 2 Gew.-%, bezogen auf die Polymerphase, wobei die Konzentration der ionischen Emulgatoren durchweg zwischen 0.01 und 0.05 Gew-% liegt. Dem Fachmann sind Einflüsse der Rührgeschwindigkeiten sowie Konzentrationen und Viskositäten der beiden Phasen auf die Partikelgröße bekannt. Zur Realisierung der bevorzugten Partikelgrößen von 0,5-10 µm sind Rührgeschwindigkeiten von 1500-2000 Umdrehungen/Minute erforderlich, wobei herkömmliche Zweiblatt-Propellerrührer zum Einsatz kommen.

Als Magnetpartikel, die während des Prozesses in die Polyvinylalkoholmatrix eingekapselt werden, können grundsätzlich solche ferro- oder superparamagnetischen Kolloide verwendet werden, die eine entsprechende Partikelgröße aufweisen und in der Regel über eine magnetische Sättigung von 50-400 Gauss verfügen. Ein weitere Forderung, die die Magnetpartikel erfüllen müssen, ist die Dispergierbarkeit in der wäßrigen Polymerphase, in der der Polyvinylalkohol vorliegt. Bei der anschließenden Emulsion in der organischen Phase werden die Magnet-Kolloide dann simultan in den Polymertröpfchen eingeschlossen.

Als magnetische Kolloide kommen vorzugsweise Magnetite mit Partikelgrößen von 10-200 nm in Frage. Solche Substanzen sind z.B. unter der Handelsbezeichnung Bayferrox oder Ferrofluidics im Handel erhältlich. Da die Herstellung solcher Kolloide allgemeiner Stand der Technik ist, können die Magnetteilchen auch nach den bekannten Verfahren, wie z.B. von Shinkai et al., Biocatalysis, Vol. 5, 1991, 61, Reimers und Khalafalla, Br. Patent 1,439,031 oder Kondo et al., Appl. Microbiol. Biotechnol., Vol 41, 1994, 99, beschrieben, hergestellt werden. Die Konzentrationen der Kolloide in der Polymerphase liegen, jeweils bezogen auf diese Phase, in der Regel zwischen 4 und 14 Vol-% bei den Kolloiden, die herstellungsbedingt bereits als wäßrige Kolloide vorliegen, und 0.3-2 Gew-% bei den Festsubstanzen. Für die Herstellung werden die magnetischen Kolloide der Polymerphase direkt zugemischt. Um eine feindisperse, gleichmäßige Verteilung der Partikel zu gewährleisten, ist ein kurzzeitiges Vermischen der wäßrigen Dispersion mittels eines hochtourigen Dispergierwerkzeuges (Ultra-Turrax) mit anschließender Ultraschallbehandlung förderlich. Die zur Herstellung der Magnetpartikel benötigte Polymerphase besteht in der Regel aus 2.5-10 Gew-% Polyvinylalkohol-Lösung.

Die erfindungsgemäß zum Einsatz kommenden Polyvinylalkoholpartikel sollten nicht porös sein. Vorzugsweise kommen daher Polymerkonzentrationen von 2.5-5 Gew-% und Molmassen von > 50000 g/mol zum Einsatz. Ein weiterer Faktor, mit dem eine Porosität der Magnetpartikel verbunden wird, ist die Wahl des Vernetzers sowie dessen Konzentration. Durch eine Vernetzung der Partikel bis zu 10% werden praktisch nicht poröse Partikel erhalten. Als Vernetzer kommen prinzipiell alle wasserlöslichen mit den Hydroxyl-Gruppen des Polyvinylalkohols reagierende, bifunktionelle Verbindungen wie z.B. Aldehyde, Säurechloride oder Divinylsulfon in Frage. Vorzugsweise wird Glutaraldehyd unter Säurekatalyse als Vernetzer verwendet, da diese Substanz bereits innerhalb weniger Minuten mit dem Polymeren unter Bildung festvernetzter Partikel abreagiert. Bei den übrigen Substanzen sind ein bis zwei Stunden Reaktionszeit erforderlich. Die Vernetzerkonzentrationen liegen, bezogen auf die wäßrige Polymerphase, in der Regel zwischen 0.2 und 1 Vol -% und für Glutaraldehyd zwischen 2 und 7 Vol-%. Glutaraldehyd wird durchweg in Form einer 6-25 % igen wäßrigen Lösung eingesetzt.

Für die Herstellung der Magnetpartikel wird im allgemeinen zunächst die 20-25fache Volumenmenge einer organischen Phase, vorzugsweise handelsübliches Pflanzenöl, vorgegeben, in der anschließend die Polymer-Magnet-Kolloid-Mischung unter Rühren suspendiert wird.

Aus der Suspension kann dann das magnetische Polyvinylalkoholträgermaterial nach den dem Fachmann an sich bekannten Methoden, beispielsweise durch Filtration und Waschen gewonnen werden.

Die erfindungsgemäßen Trägermaterialien auf Basis von Polyvinylalkohol werden durch Umsetzung dieses magnetischen Polyvinylalkoholträgermaterials, vorzugsweise in Partikelform, mit einer organischen Silanverbindung der Formeln (I), (II) oder (III) hergestellt.

Formel I

X_{q}-Si-(OR)_{4-q} (I)

worin
q eine ganze Zahl von 0-3,
R, gleich oder verschieden, für Wasserstoff, einen Alkylrest, vorzugsweise mit C₁-C₆, besonders bevorzugt mit C₁-C₂, einen Arylrest, vorzugsweise einen Phenylrest und
X, gleich oder verschieden, für Wasserstoff, einen Alkylrest, vorzugsweise C₁-C₂, einen Arylrest, vorzugsweise einen Phenylrest, oder ein Halogen, vorzugsweise Chlor, stehen,
oder Formel (II),

(Y-R¹)_{q}-Si(OR)_{4-q} (II)

worin
R, q die für die allgemeine Formel (I) angegebene Bedeutung haben,
R¹ für einen Alkylenrest mit C₁-C₆, vorzugsweise einen Ethylen- oder Propylenrest, steht,
Y für eine Aminogruppe, eine Dialkylaminogruppe, vorzugsweise eine Dimethyl-, Diethyl-aminogruppe, SH, eine Epoxidgruppe, eine Vinylgruppe, vorzugsweise eine - CR₂ = CR³₂-Gruppe mit R² bzw. R³, gleich oder verschieden, für Wasserstoff, einen Alkylrest, vorzugsweise mit C₁-C₂, einen Arylrest, vorzugsweise einen Phenylrest, oder einen Acrylsäurerest steht,
oder einer polymeren Silanverbindung mit der wiederkehrenden Einheit der allgemeinen Formel (III) 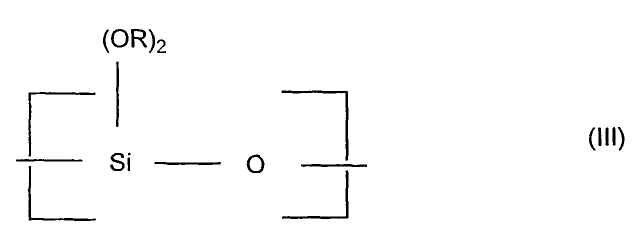
worin R die in der allgemeinen Formel I angegebene Bedeutung hat, vorzugsweise für einen Methylrest steht, einsetzt.

Ein weiterer Gegenstand ist auch ein Verfahren zum wiederholten Silanisieren der bereits silanisierten Oberflächen der magnetischen Trägermaterialien auf Basis von Polyvinylalkohol durch Umsetzung mit organischen Silanverbindungen der allgemeinen Formel II, um weitere funktionelle Gruppen einzuführen, die anschließend mit bekannten Affinitätsliganden umgesetzt werden können. Diese Affinitätsliganden können mit Biomolekülen koppeln und zu deren Isolierung und Identifizierung eingesetzt werden.

Als Affinitätsliganden können grundsätzlich sämtliche in der Affinitätschromatographie verwendeten Liganden gekoppelt werden. Beispiele hierfür, die auch aus praktischer Sicht interessante Perspektiven eröffnen, sind:

Protein A, Protein G, Protein L, Streptavidin, Biotin, Heparin, Antikörper, Serum Albumin, Gelatine, Lysin, Concanavalin A, Oligosaccharide, Oligonucleotide, Polynucleotide, proteinbindende Metallionen, Lektine oder Enzyme. Die speziellen Auftrennungen, die sich mit solchen Affinitätsmatrizes durchführen lassen, sind allgemeiner Stand der Technik. Bezüglich der Einzelheiten dieser an sich bekannten Verfahren wird auf die Ausführungen im J. of chromatography, Vol. 510, 1990, verwiesen. In der Regel ist all diesen Auftrennungen, die mit Hilfe der erfindungsgemäßen, oberflächenmodifizierten, magnetischen, silanisierten Trägermaterialien durchgeführt werden gemeinsam, daß sie ohne größeren Aufwand innerhalb von 2-5 Minuten durchgeführt werden können.

Ein weiterer, interessanter Bereich, der mit dem Einsatz magnetischer Trägermaterialien, vorzugsweise magnetische Partikel, abgedeckt wird, ist der Bereich der Diagnostik, im besonderen der Bereich des Immunoassays. Das grundlegende Prinzip besteht darin, spezifische Substanzen quantitativ zu erfassen. In der Praxis geschieht diese spezifische Bindung in der Regel über einen immobilisierten Antikörper. Die erfindungsgemäß ggf. mehrfach silanisierten magnetischen Trägermaterialien bieten hier nun eine hervorragende Basis, für Immunoassays eingesetzt zu werden. Dazu werden in der bekannten Art und Weise Antikörper gegen bestimmte, für die Diagnose relevante Antigene an die Magnetpartikel chemisch gebunden. Beispiele solcher Antikörper sind: anti-Insulin, anti-Thyroxin, Antikörper gegen das Thyroid-stimulierende Hormon (TSH), Antikörper gegen das Thyroid bindende Globulin, anti-Cortison, anti-Ferritin, anti-Chorionic Gonadotropin, anti-carcinogen-Embryonales-Antigen (CEA), anti-Progesteron, anti-Testosteron, anti-Estradiol, anti-Prolactin, anti-Human-Growth-Hormon, anti-Digoxin, anti-ß2-Microglobulin, anti-α2-Macroglobulin, anti-Vitamin B12, anti-Faktor VIII, Antikörper gegen Zelloberflächen-Antigene (sogenannte Anti CDx-Antikörper) oder anti-AFP. Die Inkubationzeiten der erfindungsgemäß mit Antikörper-gekoppelten, magnetischen Trägermaterial Proben beträgt in der Regel 2-5 Minuten. Nach magnetischer Abtrennung des Ziel-Antigens durch Bildung des hochspezifischen, trägergebundenen Antikörper-Antigenkomplexes erfolgt der Nachweis entweder unter Verwendung eines weiteren markierten Antikörpers oder nach Ablösen des Antigens unter Elutionsbedingungen direkt photometrisch. Außer Antikörper können auch andere Substanzen an die erfindungsgemäßen, magnetischen silanisierten Trägermaterialien, vorzugsweise in Partikelform, gekoppelt werden und zur Detektierung bestimmter Substanzen genutzt werden. Eine solche Substanz ist z. B. 3-Aminophenylboronsäure, die zur Detektion des Blutzuckergehaltes verwendet werden kann. Zur Immobilisierung des Liganden wird dieser mit dem Polyvinylalkohol-Träger, dessen OH-Funktionen zur Ligandenbindung mit einem DiIsocyanat aktiviert wurden, umgesetzt. Für die Umsetzung werden in Regel 15-30 mg 3-Aminophenylboronsäure pro 100 mg magnetischem Träger eingesetzt. Die Analyse des Blutzuckergehaltes geschieht über das im Blut vorhandene glycosylierte Hämoglobin, das sich spezifisch an die Boronsäure-Liganden bindet. Durch anschließende Elution der gebundenen glycosylierten Fraktion von der Matrix kann diese quantitativ mittels photometrischer Methoden analysiert werden. Die Methode kann daher besonders vorteilhaft für routinemaßige Analysen eingesetzt werden.

Ebenfalls Gegenstand der Erfindung ist daher ein Verfahren zur Isolierung und/oder Reinigung eines biologischen Materials durch
- Inkontaktbringen einer Probe, die das biologische Material in einer Flüssigkeit enthält, mit den erfindungsgemäßen, ggf. oberflächenmodifizierten, magnetischen, silanisierten Partikeln auf Basis von Polyvinylalkohol unter Bedingungen, bei denen das biologische Material an die Partikeloberfläche bindet, und
- Abtrennung des biologischen Materials von der Flüssigkeit.

Unter biologischen Materialien werden, wie bereits dargelegt, Materialien auf partikulärer oder molekularer Basis verstanden. Hierzu gehören insbesondere Zellen, z.B. Viren und Bakterien aber auch humane und tierische isolierte Zellen, wie Leukozyten, sowie immunologisch aktive nieder-und hochmolekulare chemische Verbindungen, wie Antigene, Antikörper und Nucleinsäuren. Besonders bevorzugt sind Nucleinsäuren, z.B. DNA oder RNA, ganz besonders bevorzugt DNA, die mit den erfindungsgemäßen, silanisierten Trägermaterialien auf Basis von Polyvinylalkohol selektiv selbst aus größer Verdünnung isoliert werden können.

Proben im Sinne der Erfindung sind beispielsweise klinische Proben, wie Blut, Serum, Mundspülflüssigkeit, Urin, Zerebralflüssigkeit, Sputum, Stuhl, Punktate und Knochenmarkproben. Die Probe kann auch aus dem Bereich der Umweltanalytik, der Lebensmittelanalytik oder der molekularbiologischen Forschung, z.B. aus Bakterienkulturen, Phagenlysaten und Produkten von Amplifikationsverfahren, z.B. wie in der PCR, stammen.

Mit dem erfindungsgemäßen Verfahren kann natives oder modifiziertes biologisches Material isoliert werden. Unter nativem biologischem Material wird Material verstanden, dessen Struktur gegenüber den natürlich vorkommenden biologischen Materialien nicht irreversibel verändert wurde. Dies schließt jedoch nicht die Modifizierung anderer Bestandteile der Probe aus. Sollen beispielsweise Zellen isoliert werden, so kann zwar das die Zellen umgebende Medium modifiziert sein, nicht jedoch die Zellen als solche. Sollen Nucleinsäuren isoliert werden, so sollen auch diese in der nativen Form, d h. nicht geschnitten oder durch Ankoppelung reaktiver Gruppen modifiziert sein. Der Begriff natives biologisches Material umfaßt daher insbesondere biotinylierte Nucleinsäuren nicht. Beispiele für native biologische Matertalien sind Phagen-DNA oder zelluläre Nucleinsäuren aus Blut.

Modifizierte biologische Materialien umfassen Materialien, die nicht in der Natur vorkommen, z. B. Nucleinsäuren, die durch Anheftung reaktiver, nachweisbarer oder zur Immobilisierung befähigenden Gruppen modifiziert sind, z.B. biotinylierte Nucleinsäuren.

In bestimmten Fällen kann die Probe ohne Vorbehandlung in dem erfindungsgemäßen Isolierungsverfahren eingesetzt werden. In vielen Fällen sollte die Probe jedoch durch eine geeignete Methode aufgeschlossen und das in der Probe enthaltende biologische Material freigesetzt werden. Verfahren zum Aufschluß von Proben sind dem Fachmann bekannt und können chemischer, enzymatischer oder physikalischer Natur sein. Auch eine Kombination dieser Verfahren ist möglich.

Hierbei können verschiedene Methoden für unterschiedliche Mikroorganismen besser geeignet sein, aber jede der nachstehend aufgeführten Methoden ist prizipiell geeignet:
Lyse mit Hilfe von Detergenzien, wie z.B. SDS, LiDS oder Sarkosyl in geeigneten Puffern, die Verwendung von Chaotropen, wie beispielsweise Guanidinhydrochlorid (GHCl), Guanidinthiocyanat (GTC), Natriumiodid (Nal), Natrium-perchlorat etc., mechanisches Auseinanderreißen, wie z.B. mittels einer französischen Presse, Ultraschall, Mahlen mit Glaskugeln, Aluminium oder in flüssigem Stickstoff, enzymatische Lyse, beispielsweise mit Lysozym, Proteinasen, Pronasen oder Cellulasen oder einem anderen der käuflich erhältlichen Enzyme für die Lyse, Lyse der Zelle mittels Bakteriophagen oder Virusinfektion, Gefriertrocknen, Osmotischer Schock, Mikrowellenbehandlung, Temperaturbehandlung, beispielsweise Erwärmen oder Kochen oder Gefrieren, z.B. in Trockeneis oder flüssigem Stickstoff und Auftauen, alkalische Lyse.

Wie bereits oben erwähnt, stellen alle der vorstehenden Verfahren Standard-Techniken zur Lyse dar, die aus dem Stand der Technik hinlänglich bekannt sind und jedes der Verfahren oder deren Kombination kann eingesetzt werden.

So ist eine Kombination aus Chaotropen und Detergenzien für die Lyse von Bakterienzellen besonders effektiv. Ein beispielhaftes, geeignetes Agens für die Lyse beinhaltet daher ein Chaotrop, wie z.B. GTC oder GHCl und ein Detergenz, wie z.B. SDS oder Sarkosyl. Diese Agentien zur Lyse können in wäßriger Lösung oder in einer Pufferlösung, d.h. als sogenannter Lysepuffer vorliegen. Als Puffer kann jeder geeignete Puffer, wie beispielsweise Tris, Bicin, Tricin oder Phosphatpuffer eingesetzt werden. Alternativ dazu kann das Lyseagens auch getrennt zugegeben werden. Geeignete Konzentrationen und Mengen der Lyseagentien variieren in Abhängigkeit von dem betreffenden System, Art der Zellen, etc. und können durch den Fachmann bestimmt werden, wobei beispielsweise Konzentrationen im Bereich von 2M bis 7M Chaotrop, wie z.B. GTC, GHCl oder Nal oder Natriumperchlorat, 0,1 M bis 1 M alkalische Agentien, wie z.B. NaOH, und 0,1 bis 50 Gew.-% (Gewicht/Volumen) Detergenzien verwendet werden können. Ein Beispiel eines solchen Lysepuffers beinhaltet daher eine wäßrige Lösung aus 4 M GTC und 1 % (Gewicht/Volumen) Sarkosyl.

Für verschiedene Lysesysteme können verschiedene Inkubationsbedingungen geeignet sein, die aus dem Stand der Technik bekannt sind. Für einen Detergenzien und/oder Chaotrope enthaltenden Lysepuffer beispielsweise kann die Inkubation bei Raumtemperatur oder bei erhöhter Temperatur, beispielweise 37 bis 65 °C erfolgen. Gleichermaßen kann auch die Inkubationszeit variiert werden, von wenigen Minuten, beispielsweise 5 Minuten bis zu Stunden, beispielsweise 1 bis 2 Stunden. Im Fall des GTC/Sarkosyl Lysepuffers und Bakterienzellen hat sich beispielsweise eine Inkubation bei 65 °C für 10 bis 20 Minuten bewährt, die aber bei Bedarf auch variiert werden kann. Für die enzymatische Lyse, beispielsweise mittels Proteinkinase K etc., können längere Behandlungszeiten, beispielsweise über Nacht erfoderlich sein.

Zur Isolierung von Nucleinsäuren aus biologischem Material ist im Fall der modifizierten Nucleinsäuren eine Bindung über die Gruppen der Nucleinsäuren möglich, die die Modifizierung darstellen, z.B. Biotin über die Bindung an mit Streptavidin modifizierten Oberflächen. Insbesondere bei Nucleinsäuren ist jedoch der Fall der direkten Bindung von Nucleinsäuren an den Träger bevorzugt, unter anderem deshalb, weil sich eine Modifizierung der Nucleinsäuren erübrigt und schon native Nucleinsäuren gebunden werden können. Erfindungsgemäß gelingt dies durch den Einsatz der silanisierten, magnetischen Trägermaterialien auf Basis von Polyvinylalkohol selektiv und effizient, selbst wenn die Proben die Nucleinsäuren in hoher Verdünnung und/oder in Mischung mit weiteren biologischen Materialien enthalten. Die Bindung nativer Nucleinsäuren an die erfindungsgemäßen Trägermaterialien kann analog zu Verfahren des Standes der Technik erfolgen.

Bevorzugt erfolgt sie in Gegenwart chaotroper Salze, wobei die Konzentration dieser Salze zwischen 2 und 8 mol/l beträgt, bevorzugt 4 bis 6 mol/l. Bei chaotropen Salzen handelt es sich z B. um Natriumjodid, Natriumperchlorat, Guanidininiumthiocyanat, Guanidiniumisothiocyanat oder Guanidiniumhydrochlorid. Die Bindung ist jedoch nicht auf diese Verbindungen beschränkt.

Zum Isolieren wird die Probe mit dem Trägermaterial, vorzugsweise den Partikeln, in Kontakt gebracht und für eine für die Bindung ausreichende Zeit inkubiert. Für Nucleinsäuren können Inkubationszeiten zwischen 10 Sekunden und 30 Minuten zweckmäßig sein.

Zur Isolierung von Nucleinsäuren werden bevorzugt silanisierte, magnetische Partikel verwendet, die perl- oder kugelförmig sind und eine Partikelgröße im Bereich von 0,2 bis 50 µm, vorzugsweise 0,5 bis 5 µm, mit sehr enger Größenverteilung aufweisen.

Nach der Inkubation erfolgt die Abtrennung des biologischen Materials, vorzugsweise der Nucleinsäuren von der Probenflüssigkeit. Dies wird allgemein durch die Separation der an die erfindungsgemäßen magnetischen Partikel gebundenen Nucleinsäuren mit Hilfe eines Magnetfeldes errreicht. Beispielsweise können die Magnetpartikel an die Wand des Gefäßes, in welchem die Inkubation stattgefunden hatte, gezogen werden. Daraufhin kann die Flüssigkeit mit den Inhaltsstoffen der Probe, die nicht an die magnetischen Partikel gebunden wurden, entfernt werden. Diese Entfernung hängt von der Art des Gefäßes ab, in dem die Inkubation stattgefunden hat. Geeignete Verfahrensschritte zum Entfernen der Flüssigkeit sind z.B. Abpipettieren oder Absaugen der Flüssigkeit.
Da das erfindungsgemäße, magnetische, silanisierte Trägermaterial sehr selektiv Nucleinsäuren aus biologischen Proben bindet, ohne daß es zu einer Kontamination des Trägermaterials mit anderen biologischen Materialien kommt, können die Waschschritte beträchtlich reduziert werden.

Falls gewünscht, können die beladenen magnetischen Partikel gewünschtenfalls ein- oder mehrmal mit einer Waschlösung gereinigt werden. Die Waschlösung wird so gewählt, daß eine Ablösung des biologischen Materials, z.B. der Nucleinsäuren, von der Partikeloberfläche möglichst nicht stattfindet, jedoch noch vorhandene Verunreinigungen möglichst gut weggewaschen werden. Dieser Waschschritt findet bevorzugt durch Inkubation der Waschlösung mit den beladenen Partikeln statt, wobei bevorzugt eine Resuspension der Partikel vorgenommen wird, z.B. durch Schütteln oder Anlegung eines nicht mit dem ersten Magnetfeld identischen Magnetfeldes. Die verunreinigte Waschlösung wird bevorzugt genauso entfernt wie die nach der Isolierung der Nucleinsäuren verbleibende Probenflüssigkeit.

Als Waschlösung kann jeder herkömmliche Waschpuffer oder jedes andere, geeignete Medium verwendet werden. Im allgemeinen werden Puffer mit niedriger bis moderater lonenstärke bevorzugt, wie z.B. 10 mM Tris-HCl bei pH 8,0/10 mM NaCl. Andere Standardmedien zum Waschen, z.B. alkoholhaltige Medien, ebenfalls eingesetzt werden, wie z.B. 70 %iges Ethanol.
Die Verwendung magnetischer Partikel ermöglicht einfache Waschschritte einfach durch die magnetische Aggregation der Partikel, Entfernen des Nucleinsäurebindenden Mediums, Zusatz des Waschmediums und Regaggregation der Partikel, so oft es erforderlich ist.

Nach dem Verfahren der Nucleinsäure-Isolierung und jedem ggf. gewünschten Waschschritt kann der die Nucleinsäure tragende Träger in jedes geeignete Medium, z.B. Wasser oder einen Puffer mit niedriger lonenstärke transferiert, z.B. resuspendiert oder eingetaucht werden.

Im Anschluß an den letzten Waschschritt kann ein kurzer Trocknungsschritt der magnetischen Partikel im Vakuum oder durch Ausdampfen (lassen) der Flüssigkeit vorgenommen werden, wobei auch eine Vorbehandlung mit Aceton möglich ist.
Der Fachmann versteht, daß die vorstehend beschriebenen Schritte des Waschens und Trocknens nicht nur bei der Reinigung und/oder Isolierung von Nucleinsäuren durchgeführt werden können, sondern auch für die Reinigung und/oder Isolierung der anderen, vorstehend genannten biologischen Materialien geeignet sind.

In Abhängigkeit von dem Träger und der Art einer nachfolgenden Aufarbeitung kann es wünschenswert sein, die Nucleinsäure von dem Träger zu lösen oder sie nicht von dem Träger zu lösen. Im Falle eines besonderen festen Trägers, wie der erfindungsgemäßen magnetischen Partikel, kann dieser in vielen Fällen direkt eingesetzt werden, wie z.B. in der PCR oder anderen Amplifikationsmethoden, ohne daß die Nucleinsäuren von dem Träger eluiert werden müssen. Desweiteren ist auch für viele DNA-Detektionsverfahren oder -Identifikationsverfahren ein Eluieren nicht erforderlich, da obwohl die DNA in zufälliger Weise mit der Oberfläche der Kügelchen in Berührung stehen und an einer Vielzahl von Punkten durch Wasserstoffbrückenbindungen oder ionische Bindungen oder andere Kräfte gebunden sein kann, eine hinreichende Länge von DNA für die Hybridisierung mit Oligonukleotiden und zur Vervielfältigung zur Verfügung steht.

Für den Fall, daß es sich bei dem biologischen Material um native Nucleinsäuren handelt, kann die Nucleinsäure mittels eines Elutionspuffers mit niedrigem Salzgehalt von den erfindungsgemäßen Partikeln entfernt werden. Solche Puffer sind aus Analytical Biochemistry 175, 196 - 201 (1988) bekannt. Als Elutionspuffer mit niedrigem Salzgehalt werden insbesondere Puffer mit einem Salzgehalt von weniger als 0,1 mol/l eingesetzt. Besonders bevorzugt enthält der Elutionspuffer Tris HCl. Besonders geeignet ist auch entmineralisiertes Wasser.

Falls erwünscht, ist es auch möglich, die RNA von der DNA zu entfernen, was durch die Zerstörung der RNA vor dem Schritt der DNA-Separierung erreicht werden kann, beispielsweise durch Zugabe von RNAse oder von Alkali, wie z. B. NaOH.

Ein weiterer Gegenstand der Erfindung ist auch die Reinigung und/oder Isolierung von Nucleinsäuren, vorzugsweise von DNA durch den Einsatz der erfindungsgemäßen, ggf. oberflächenmodifizierten, silanisierten, magnetischen Trägermaterialien.

Das erfindungsgemäße Verfahren zur Isolierung von Nucleinsäuren kann auch im Anschluß an eine immunomagnetische Separation von Zellen aus einer Körperflüssigkeit oder einem Gewebe erfolgen. Hierzu wird die Probe mit den vorstehend beschriebenen erfindungsgemäßen magnetischen Partikeln, an welche ein Antikörper gegen ein Antigen auf der Zelle immobilisiert ist, z.B. unter Schütteln inkubiert. Nach Anlegen eines Magnetfeldes erfolgen ein oder mehrere Waschschritte mit einer salzhaltigen Waschlösung. Man erhält so Partikel, an welche die gewünschten Zellen gebunden sind. Schließlich werden die gebundenen Zellen in einem salzhaltigen Puffer resuspendiert. In einer bevorzugten Ausführungsform ist dieser salzhaltige Puffer eine chaotrope Salzlösung, so daß die in der Zelle vorhandenen Nucleinsäuren aus den Zellen freigesetzt werden.

Durch Kombination der oben beschriebenen erfindungsgemäßen Isolierung von Zellen mit der ebenfalls beschriebenen erfindungsgemäßen Isolierung von Nucleinsäuren, bevorzugt in ihrer nativen Form, an den erfindungsgemäßen magnetischen Trägermaterialien, vorzugsweise in Partikelform, ergibt sich ein besonders vorteilhaftes Verfahren zur Isolierung von Nucleinsäuren aus zellhaltigen Proben. Vorteil dieser Ausführungsform ist deren Einfachheit, hohe Sensitivität und Selektivität und deren leichte Automatisierbarkeit.

Die als Folge der erfindungsgemaßen Verfahren isolierten biologischen Materialien können nun in beliebiger Weise weiter verwendet werden. Beispielsweise können sie als Substrat für verschiedene enzymatische Reaktionen verwendet werden. Im Falle der Nucleinsäuren seien als Beispiel die Sequenzierung, die radioaktive oder nichtradioaktive Markierung, die Amplifikation einer oder mehrerer in ihr enthaltender Sequenzen, die Transkription, die Hybridisierung mit markierten Sondennucleinsäuren, die Translation oder die Ligation genannt. Ein Vorteil des erfindungsgemäßen Verfahrens ist, daß die Abtrennung des biologischen Materials, insbesondere der Nucleinsäuren von der Flüssigkeit sehr einfach ist.
Im folgenden wird die Erfindung anhand von Beispielen erläutert.

### Beispiel 1:

### 1a) Silanisierung:

In einem Reaktionsgefäß wurden 55 mg superparamagnetischer Polyvinylalkoholpartikel mit einer Teilchengröße von 0,5 bis 1 µm in 2,5 ml einer wäßrigen Lösung von Natriumacetat (Konzentration 100 mM, pH = 5,5) suspendiert und in einem Ölbad auf eine Temperatur von 90 °C erhitzt. Anschließed wurden 25 µl Tetramethoxysilan (Fluka, Deisenhofen) in das Reaktionsgefäß gegeben und das Reaktionsgemisch für 2 Stunden unter Rühren inkubiert. Nach Ablauf der zwei Stunden wurde das magnetische Trägermaterial dreimal mit jeweils 30 ml destiliertem Wasser gewaschen und in Wasser resuspendiert.

### 1b) Isolierung von DNA:

Die Isolierung von 1 µg nativer lambda DNA (Fischer Biotech, Nidderau) wurde unter Verwendung von 50 µg gemäß Beispiel 1a) silanisierter Partikel durchgeführt.

50 µg der Partikel wurden in 250 µl einer 7M NaClO₄ suspendiert. Die so erhaltene Suspension wurde dann in ein Mikroliter-Gefäß mit 1 µg lambda DNA in 100 µl Wasser gegeben. Der Inhalt dieses Gefäßes wurde dann gemischt und für 5 Minuten bei Raumtemperatur inkubiert. Nach der Inkubation wurden die Partikel mit Hilfe eines magnetischen Separators (chemagen AG, Baesweiler, BRD) von der Lösung abgetrennt. Der Überstand wurde verworfen und die Partikel wurden jeweils dreimal mit 500 µl einer 70 %igen Ethanol-Waschlösung gewaschen, wobei die Partikel nach jedem Waschvorgang magnetisch abgetrennt und der Überstand verworfen wurde. Im Anschluß an den letzten Waschvorgang wurden die Partikel für 5 Minuten an der Luft getrocknet.

Anschließend wurden die Partikel in dem Reaktionsgefäß in 35 µl einer 10 mM Triaminomethanhydrochlorid (Tris-HCl) -Lösung (pH 8.0) resuspendiert und bei einer Temperatur von 55 °C auf einem Wasserbad für 10 Minuten unter gelegentlichem Schütteln inkubiert. Abschließend wurden die Partikel im magnetischen Separator (chemagen AG, Baesweiler, BRD) von dem Überstand abgetrennt und der Überstand wurde in ein sauberes Mikroliter-Gefäß überführt. 15 µl des Eluats wurden dann auf ein gefärbtes 1.5%-iges Agarosegel (Gelstar®, FMC Corporation) geladen. Die Elektrophorese wurde dann unter Verwendung von TBE-Puffer (0,1M, pH 8,4, LifeTechnologies, Karlsruhe) durchgeführt.

Die Auswertung der Gel-Elektrophorese zeigt ein starkes, deutlich detektierbares Signal für die mit Hilfe der erfindungsgemäßen silanisierten, magnetischen Trägermaterialien isolierte DNA.

### Beispiel 2:

### Vergleichsbeispiel

### Isolierung von DNA:

Die Isolierung von 1 µg nativer lambda DNA (Fischer Biotech, Nidderau) wurde unter Verwendung von 50 µg der magnetischen Polyvinylalkohol-Partikel gemäß Beispiel 1a) durchgeführt, die nicht silanisiert wurden.

50 µg der Partikel wurden jeweils in 250 µl einer 7M NaClO₄ suspendiert. Die so erhaltene Suspension wurden dann in ein Mikroliter-Gefäß mit 1 µg lambda DNA in 100 µl Wasser gegeben. Der Inhalt dieses Gefäßes wurde dann gemischt und für 5 Minuten bei Raumtemperatur inkubiert. Nach der Inkubation wurden die Partikel mittels eines magnetischen Separators (chemagen AG, Baesweiler, BRD) von der Lösung abgetrennt. Der Überstand wurde verworfen und die Partikel wurden jeweils dreimal mit 500 µl einer 70 %igen Ethanol-Waschlösung gewaschen, wobei die Partikel nach jedem Waschvorgang magnetisch abgetrennt und der Überstand verworfen wurde. Im Anschluß an den letzten Waschvorgang wurden die Partikel für 5 Minuten an der Luft getrocknet.
Anschließend wurden die Partikel in dem Reaktionsgefäß in 35 µl einer 10 mM Tris-HCl-Lösung (pH 8.0) resuspendiert und bei einer Temperatur von 55 °C auf einem Wasserbad für 10 Minuten unter gelegentlichem Schütteln inkubiert. Abschließend wurden die Partikel im magnetischen Separator von dem Überstand abgetrennt und der Überstand wurden in ein sauberes Mikroliter-Gefäß überführt. 15 µl des Eluats wurden dann auf ein gefärbtes 1.5%-iges Agarosegel (Gelstar®, FMC Corporation) geladen. Die Elektrophorese wurde dann unter Verwendung von TBE-Puffer (0,1 M, pH 8,4, Life Technologies, Karlsruhe) durchgeführt.

Die Auswertung der Gel-Elektrophorese zeigt ein schwaches, kaum zu detektierendes Signal für die mit Hilfe der unsilanisierten Trägermaterialien isolierte DNA.

### Beispiel 3

Zu 10 µl humanem, mit Ethylendiamintetraessigsäure (EDTA-) behandeltem Vollblut werden 2 µl Proteinase K (20 mg/ml in Wasser, Appligene, Heidelberg) und anschließend 100 µl Lyse-Puffer (1 M Guanidinium-Hydrochlorid, 10 mM Tris-HCl, 6% Triton X-100, pH 7) zugegeben. Die Mischung wird 15 Minuten bei 45 °C inkubiert und anschließend mit 600 µg der silanisierten, magnetischen Partikel gemäß Beispiel 1a) sowie 300 µl Bindungspuffer (90 % Ethanol, 100 mM Tris-HCl, pH 7) versetzt. Nach 10 minütiger Inkubation der zuvor gut durchmischten Suspension bei Raumtemperatur werden die magnetischen Partikel in einem magnetischen Separator (chemagen AG, Baesweiler, BRD) magnetisch abgetrennt und der Überstand verworfen. Die an den Partikeln gebundene DNA wird dreimal mit jeweils 1 ml 80 %igem Isopropanol gewaschen und dann 10 Minuten an der Luft getrocknet, um restliches Isopropanol zu entfernen. In 30 µl TBE-Puffer wird resuspendiert und 10 Minuten bei 65 °C inkubiert. Nach magnetischer Separation wird die eluierte DNA-Lösung abgetrennt und die DNA einer Amplifikation mittels der PCR-Technologie unterworfen.

### Beispiel 4: Isolierung von genomischer DNA aus 5 ml Vollblut

5 ml Vollblut (EDTA-stabilisiert) werden in einem 50 ml Reaktionsgefäß mit 6,25 ml Lyse-Puffer (1,2 M Guanidin-Hydrochlorid, 30 mM Tris-HCl, pH 7, 30 mM EDTA, 10% Tween 20® und 1% Triton X-100® von Fluca) durchmischt und 5 Minuten bei Raumtemperatur inkubiert. Anschließend werden 600 µl der modifizierten Partikel aus Beispiel 1 und 18 ml Bindungspuffer (60% Ethanol, 1,2 M NaClO₄, 0,2 M Natriumacetat) zugesetzt. Nach fünfminütiger Inkubation bei Raumtemperatur wird der Überstand nach magnetischer Separation verworfen. Die Partikel werden anschließend mit 30 ml Waschpuffer A (30% Ethanol, 1,1 M NaClO₄, 0,15 M Natriumacetat), dann zweimal mit je 30 ml 60% Ethanol und anschließend kurz mit 40 ml Wasser gewaschen. Nach Abtrennung der letzten Waschlösung wird in 1 ml 10 mM Tris-HCl eluiert durch fünfminütige Inkubation bei 55 °C. Die isolierte genomische DNA kann direkt z.B. in der PCR eingesetzt werden.

### Beispiel 5: Aufreinigung eines PCR-Amplifikationsproduktes

Ein 595 bp DNA-Fragment der malB-Region von genomischer DNA isoliert aus E.coli-Bakterien (K12) mit Hilfe eines Kits umfassend magnetische Teilchen auf Polyvinylalkohol Basis bindend Bakterien und bakterielle DNA, sogenannte Bugs'n Beads Kit (M-PVA DNA 200 Kit von chemagen AG) wird in einer PCR amplifiziert (Primer und Bedingungen entsprechend Candrian et al. Int. J. Food Microbiol. 12 (1991) 339). 50 µl des PCR-Produkt werden nach der Amplifikation mit 16 µl der Partikelsuspension aus Beispiel 1 versetzt und 100 µl Bindungspuffer (90% Ethanol, 100 mM Tris-HCl, pH 7) hinzugefügt. Nach fünfminütiger Inkubation wird magnetisch separiert und der Überstand verworfen. Es wird zweimal mit jeweils 400 µl 60%igem Ethanol gewaschen und die Partikel nach vollständiger Abtrennung der Waschlösung 8 Minuten bei geöffnetem Gefäß getrocknet. Anschließend wird in 30 µl 10 mM Tris-HCl pH 8,0 resuspendiert und nach 5 minütiger Inkubation bei 55 °C der Überstand mit dem aufgereinigten DNA-Amplifikat von den magnetischen Teilchen abgetrennt und in ein neues Gefäß überführt.

### Beispiel 6: Isolierung von Plasmid-DNA aus E.coli-Bakterien

1,5 ml Bakterienkultur (E.coli mit Plasmid pUC18, Übernachtkultur) werden in einem 1,5 ml Reaktionsgefäß zunächst bei 6000 g für zwei Minuten zentrifugiert und der Überstand verworfen. Mit 100 µl Resupensions-Puffer (TE Puffer, pH 8) wird das Bakterienpellet resuspendiert, 100 µl Lyse-Puffer (0,1 M NaOH, 0,2% Natriumdodecylsulfat (SDS)) zugegeben und 5 Minuten bei Raumtemperatur inkubiert. Anschließend werden 140 µl Neutralisationspuffer (4 M Guanidinhydrochlorid, 0,5 M Kaliumacetat, pH 4,2) und 20 µl der modifizierten Partikel aus Beispiel 1 zugesetzt. Nach fünfminütiger Inkubation bei Raumtemperatur wird der Überstand nach magnetischer Separation verworfen. Die Partikel werden anschließend mit 500 µl Waschpuffer A (30% Ethanol, 1,1 M NaClO₄, 0,15 M Natriumacetat), dann mit 500 µl 70% Ethanol gewaschen. Nach Abtrennung der letzten Waschlösung werden die Partikel an der Luft 6 Minuten getrocknet und in 50 µl 10 mM Tris-HCl eluiert durch fünfminütige Inkubation bei 55 °C.

### Beispiel 7: Isolierung von viraler DNA (aus Hepatitis B Viren, HBV) aus Seren

200 µl Serum eines Hepatitis B positiven Patienten werden in einem 1,5 ml Reaktionsgefäß mit 200 µl Lyse-Puffer (1,2 M Guanidin-Hydrochlorid, 30 mM Tris-HCl, pH 7, 30 mM EDTA, 10% Tween 20 und 1 % Triton X-100) durchmischt und 5 Minuten bei Raumtemperatur inkubiert. Anschließend werden 30 µl der modifizierten Partikel aus Beispiel 1 und 600 µl Bindungspuffer (60% Ethanol, 1,2 M NaClO₄, 0,2 M Natriumacetat) zugesetzt. Nach fünfminütiger Inkubation bei Raumtemperatur wird der Überstand nach magnetischer Separation verworfen. Die Partikel werden anschließend mit 500 µl Waschpuffer A (30% Ethanol, 1,1 M NaClO₄, 0,15 M Natriumacetat), dann mit 500 µl 70% Ethanol und anschließend kurz mit 600 µl Wasser gewaschen. Nach Abtrennung der letzten Waschlösung wird in 1 ml 10 mM Tris-HCl eluiert durch fünfminütige Inkubation bei 55 °C. Die isolierte virale DNA kann direkt z.B. in der PCR mit spezifischen Primern zur Hepatitis B Virus-Diagnostik eingesetzt werden.

## Patentansprüche

1. Magnetische Polyvinylalkoholträgermaterialien, deren Oberfläche zumindest teilweise silanisiert ist, **dadurch** erhältlich, dass man das Polyvinylalkoholträgermaterial mit einer organischen Silanverbindung umsetzt ausgewählt aus der Gruppe bestehend aus
- Silanverbindungen der allgemeinen Formel (I)
X_{q}-Si-(OR)_{4-q} (I)
worin
q eine ganze Zahl von 0 bis 3,
R, gleich oder verschieden, für Wasserstoff, einen Alkylrest, vorzugsweise mit C₁-C₆, besonders bevorzugt mit C₁-C₂, einen Arylrest, vorzugsweise ein Phenylrest
und
X, gleich oder verschieden, für Wasserstoff, einen Alkylrest, vorzugsweise C₁-C₂, einen Arylrest, vorzugsweise einen Phenylrest, oder ein Halogen, vorzugsweise Chlor, stehen;
- Silanverbindungen der allgemeinen Formel (II)
(Y-R¹)_{q}-Si(OR)_{4-q} (II)
worin
R, q die für die allgemeine Formel (I) angegebene Bedeutung haben,
R¹ für einen Alkylenrest mit C₁-C₆, vorzugsweise einen Ethylen- oder Propylenrest, steht,
Y für eine Vinylgruppe, vorzugsweise eine -CR² = CR³₂-Gruppe mit R² bzw. R³, gleich oder verschieden, für Wasserstoff, einen Alkylrest, vorzugsweise mit C₁-C₂, einen Arylrest, vorzugsweise Phenylrest, oder einen Acrylsäurerest steht;
und
- polymeren Silanverbindungen mit der wiederkehrenden Einheit der allgemeinen Formel (III) 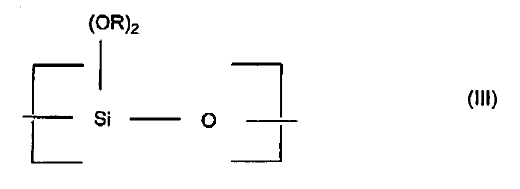 worin R die in der allgemeinen Formel I angegebene Bedeutung hat, vorzugsweise für einen Methylrest steht.

2. Polyvinylalkoholträgermaterialien nach Anspruch 1, **dadurch gekennzeichnet, dass** sie ferromagnetisch oder superparamagnetisch sind.

3. Polyvinylalkoholträgermaterialien nach Anspruch 1 oder 2, **dadurch gekennzeichnet, dass** sie als Hydrogel vorliegen.

4. Polyvinylalkoholträgermaterialien nach einem der Ansprüche 1 bis 3, **dadurch gekennzeichnet, dass** sie als perl- oder kugelförmige Partikel vorliegen.

5. Polyvinylalkohoiträgermaterialien nach Anspruch 4, **dadurch gekennzeichnet, dass** die Partikel eine Partikelgröße von 0,2 bis 50 µm, vorzugsweise 0,5 bis 5 µm, aufweisen.

6. Polyvinylalkoholträgermaterialien nach Anspruch 1, **dadurch gekennzeichnet, dass** sie als Filter oder Membran vorliegen.

7. Verfahren zur Herstellung eines oberflächenmodifizierten, magnetischen Polyvinylalkoholträgermaterials nach einem der Ansprüche 1 bis 6, **dadurch gekennzeichnet, dass** man das Polyvinylalkoholträgermaterial mit einer organischen Silanverbindung
- der allgemeinen Formel (I)
X_{q}-Si-(OR)_{4-q} (I)
worin
q eine ganze Zahl von 0-3,
R, gleich oder verschieden, für Wasserstoff, einen Alkylrest, vorzugsweise mit C₁-C₆, besonders bevorzugt mit C₁-C₂, einen Arylrest, vorzugsweise ein Phenylrest
und
X, gleich oder verschieden, für Wasserstoff, einen Alkylrest, vorzugsweise C₁-C₂, einen Arylrest, vorzugsweise einen Phenylrest, oder ein Halogen, vorzugsweise Chlor, stehen,
oder
- der allgemeinen Formel (II)
(Y-R¹)_{q}-Si(OR)_{4-q} (II)
worin
R, q die für die allgemeine Formel (I) angegebene Bedeutung haben,
R¹ für einen Alkylenrest mit C₁-C₆, vorzugsweise einen Ethylen- oder Propylenrest, steht,
Y für eine Aminogruppe, eine Dialkylaminogruppe, vorzugsweise eine Dimethyl-, Diethyl-aminogruppe, SH, eine Epoxidgruppe, eine Vinylgruppe, vorzugsweise eine -CR₂ = CR³₂-Gruppe mit R² bzw. R³, gleich oder verschieden, für Wasserstoff, einen Alkylrest, vorzugsweise mit C₁-C₂, einen Arylrest, vorzugsweise Phenylrest, oder einen Acrylsäurerest steht,
oder
- einer polymeren Silanverbindung mit der wiederkehrenden Einheit der allgemeinen Formel (III) 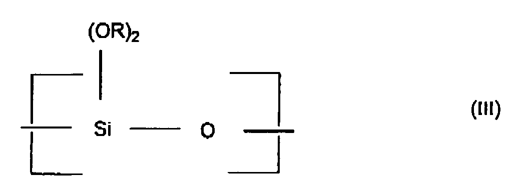 worin R die in der allgemeinen Formel I angegebene Bedeutung hat, vorzugsweise für einen Methylrest steht,
umsetzt.

8. Verfahren nach Anspruch 7, **dadurch gekennzeichnet, dass** die Umsetzung bei Temperaturen von 70 bis 150°C, vorzugsweise von 80 bis 100°C erfolgt.

9. Verfahren nach Anspruch 7 oder 8, **dadurch gekennzeichnet, dass** das Polyvinylalkoholträgermaterial, vorzugsweise die Polyvinylalkoholpartikel, im wäßrigen Medium vorzugsweise bei einem pH von 2 bis 7 zur Umsetzung suspendiert wird (werden).

10. Verfahren nach einem der Ansprüche 7 bis 9, **dadurch gekennzeichnet, dass** das Polyvinylalkoholträgermaterial, vorzugsweise die Polyvinylalkoholpartikel, zur Umsetzung in einem hydrophoben, organischen Lösungsmittel suspendiert wird (werden).

11. Verfahren nach einem der Ansprüche 7 bis 10, **dadurch gekennzeichnet, dass** die Umsetzung, vorzugsweise unter Durchmischung des Polyvinylalkoholträgermaterials und der Silanverbindung, für eine Dauer von 1 Minute bis 48 Stunden erfolgt.

12. Verfahren nach einem der Ansprüche 7 bis 11, **dadurch gekennzeichnet, dass** die OH Gruppen an der Oberfläche des Polyvinylalkoholträgermaterials mit der 1,2 bis 1,8 equivalenten Menge der Silanverbindung umgesetzt werden.

13. Verfahren nach einem der Ansprüche 7 bis 12, **dadurch gekennzeichnet, dass** das Polyvinylalkoholträgermaterial nach der Umsetzung mit einer Silanverbindung der allgemeinen Formel (I) bis (III), ggf. nach Abtrennung aus dem Reaktionsmedium und Reinigung, nochmals mit einer Silanverbindung der allgemeinen Formel (II) umgesetzt wird.

14. Verwendung von Polyvinylalkoholträgermaterialien nach den Ansprüchen 1 bis 6 oder erhältlich nach den Ansprüchen 7 bis 13 zur Isolierung und/oder Reinigung von Nucleinsäuren aus biologischen Proben, **dadurch gekennzeichnet, dass** man die Polyvinylalkoholträgermaterialien zur Immobilisierung und Abtrennung der Nucleinsäuren einsetzt.

15. Verwendung nach Anspruch 14, **dadurch gekennzeichnet, dass** die biologische Probe mit einer zur Extraktion der Nucleinsäuren geeigneten Pufferlösung versetzt wird.

16. Verwendung nach Anspruch 15, **dadurch gekennzeichnet, dass** die biologische Probe vor der Extraktion mit einem Agens, vorzugsweise RNAse oder Alkali, versetzt wird, das die Ribonucleinsäure (RNA) zerstört.

## Claims

1. A magnetic polyvinyl alcohol carrier material having an at least partially silanized surface, obtainable by causing said magnetic polyvinyl alcohol carrier material to react with an organic silane compound selected from the group consisting of
silane compounds of the general formula (I)
Xq-Si-(OR)_{4-q} (I),
in which
q is an integer from 0 to 3,
R, the same or different, stands for hydrogen, an alkyl radical containing preferably from 1 to 6 carbons and more preferably 1 or 2 carbons, an aryl radical, preferably a phenyl radical,
and
X, the same or different, stands for hydrogen, an alkyl radical containing preferably 1 or 2 carbons, an aryl radical, preferably a phenyl radical, or a halogen, preferably chlorine;
silane compounds of the general formula (II)
(Y-R¹)_{q}-Si(OR)_{4-q} (II),
in which
R, q have the meanings stated for the general formula (I),
R¹ stands for an alkylene radical containing from 1 to 6 carbons, preferably an ethylene or propylene radical,
Y stands for a vinyl group, preferably a -CR²=CR³₂ group in which R² or R³, the same or different, stands for hydrogen, an alkyl radical containing preferably 1 or 2 carbons, an aryl radical, preferably a phenyl radical or an acrylic acid radical,
and polymeric silane compounds containing recurring units of the general formula (III) 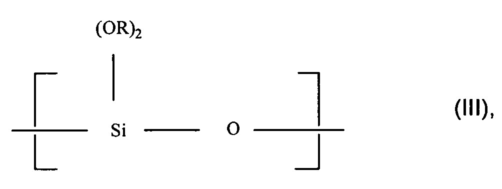 in which R has the meaning stated for the general formula (I) and preferably stands for a methyl radical.

2. A polyvinyl alcohol carrier material as defined in claim 1, **characterized in that** it is ferromagnetic or superparamagnetic.

3. A polyvinyl alcohol carrier material as defined in claim 1, **characterized in that** it exists in the form of a hydrogel.

4. A polyvinyl alcohol carrier material as defined in claim 1, **characterized in that** it exists in the form of beaded or spherical particles.

5. A polyvinyl alcohol carrier material as defined in claim 4, **characterized in that** said particles have a particle size of from 0.2 to 50 µm, preferably 0.5 to 5µm.

6. A polyvinyl alcohol carrier material as defined in claim 1, **characterized in that** it exists in the form of a filter or membrane.

7. A process for the production of a surface-modified, magnetic polyvinyl alcohol carrier material as defined in any one of claims 1 to 6, **characterized in that** the polyvinyl alcohol carrier material is caused to react with an organic silane compound selected from the group consisting of
silane compounds of the general formula (I)
X_{q}-Si-(OR)_{4-q} (I),
in which
q is an integer from 0 to 3,
R, the same or different, stands for hydrogen, an alkyl radical containing preferably from 1 to 6 carbons and more preferably 1 or 2 carbons, an aryl radical, preferably a phenyl radical, and
X, the same or different, stands for hydrogen, an alkyl radical containing preferably 1 or 2 carbons, an aryl radical, preferably a phenyl radical, or a halogen, preferably chlorine;
silane compounds of the general formula (II)
(Y-R¹)_{q}-Si(OR)_{4-q} (II),
in which
R, q have the meanings stated for the general formula (I),
R¹ stands for an alkylene radical containing from 1 to 6 carbons, preferably an ethylene or propylene radical,
Y stands for an amino group, a dialkyl amino group, preferably a dimethyl-, diethyl-amino group, SH, an epoxy group, a vinyl group, preferably a -CR²=CR³₂ group in which R² or R³, the same or different, stands for hydrogen, an alkyl radical containing preferably 1 or 2 carbons, an aryl radical, preferably a phenyl radical, or an acrylic acid radical; and
polymeric silane compounds containing recurring units of the general formula (III) 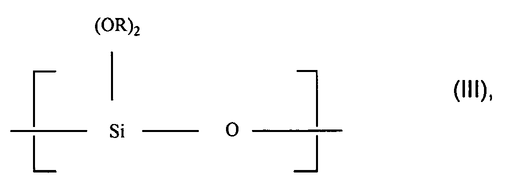 in which R has the meaning stated for the general formula (I) and preferably stands for a methyl radical.

8. A process as defined in claim 7, **characterized in that** the reaction is carried out at temperatures ranging from 70 to 150° C, preferably from 80 to 100° C.

9. A process as defined in claim 7 or claim 8, **characterized in that** the polyvinyl alcohol material, preferably in the form of polyvinyl alcohol particles, is suspended in an aqueous medium preferably having a pH of from 2 to 7 for the reaction.

10. A process as defined in any one of claims 7 to 9, **characterized in that** the polyvinyl alcohol material, preferably in the form of polyvinyl alcohol particles, is suspended in a hydrophobic organic solvent for the reaction.

11. A process as defined in any one of claims 7 to 10, **characterized in that** the reaction is carried out, preferably with stirring of the polyvinyl alcohol carrier material with the silane compound, for a period of from 1 minute to 48 hours.

12. A process as defined in any one of claims 7 to 11, **characterized in that** the OH groups on the surface of the polyvinyl alcohol carrier material are caused to react with from 1.2 to 1.8 equivalents of the silane compound.

13. A process as defined in any one of claims 7 to 12, **characterized in that** following the reaction with a silane compound of the general formulas (I) to (III), the polyvinyl alcohol carrier material, optionally after separation from the reaction medium and purification, is again caused to react with a silane compound with the general formula (II).

14. Use of a polyvinyl alcohol carrier material as defined in any one of claims 1 to 6 or as obtainable according to any one of claims 7 to 13 for the isolation and/or purification of nucleic acids from a biological specimen, **characterized in that** said polyvinyl alcohol carrier material is used for the immobilization and separation of said nucleic acids.

15. Use as defined in claim 14, **characterized in that** to said biological specimen there is added a buffer solution suitable for the extraction of nucleic acids.

16. Use as defined in claim 15, **characterized in that** prior to said extraction there is added to the biological specimen an agent, preferably an RNase or an alkali capable of destroying ribonucleic acid (RNA).

## Revendications

1. Matériaux de support magnétiques à base d'alcool polyvinylique, dont la surface est au moins partiellement silanisée, que l'on peut obtenir par le fait qu'on fait réagir le matériau de support à base d'alcool polyvinylique avec un composé de silane organique choisi parmi le groupe constitué
- de composés de silane de la formule générale (I) :
X_{q}-Si-(OR)_{4-q} (I)
dans laquelle
q est un nombre entier de 0 à 3,
R sont identiques ou différents et représentent de l'hydrogène, un radical alkyle, de préférence en C₁-C₆, particulièrement avantageusement en C₁-C₂, un radical aryle, de préférence un radical phényle,
et
X sont identiques ou différents et représentent de l'hydrogène, un radical alkyle, de préférence en C₁-C₂, un radical aryle, de préférence un radical phényle, ou un halogène, de préférence du chlore,
- de composés de silane de la formule générale (II) :
(Y-R¹)_{q}-Si(OR)_{4-q} (II)
dans laquelle
R, q ont la signification donnée pour la formule générale (I),
R¹ représente un radical alkylène en C₁-C₆, de préférence un radical éthylène ou propylène,
Y représente un groupe vinyle, de préférence un groupe -CR²=CR³₂, où R² et respectivement R³ sont identiques ou différents et représentent de l'hydrogène, un radical alkyle, de préférence en C₁-C₂, un radical aryle, de préférence un radical phényle, ou un radical d'acide acrylique,
et
- de composés de silane polymères comportant l'unité répétitive de la formule générale (III) : 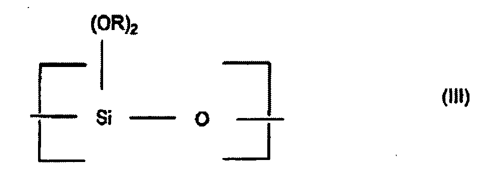 dans laquelle R, qui a la signification indiquée dans la formule générale (I), est de préférence un radical méthyle.

2. Matériaux de support à base d'alcool polyvinylique suivant la revendication 1, **caractérisés en ce qu'**ils sont ferromagnétiques ou superparamagnétiques.

3. Matériaux de support à base d'alcool polyvinylique suivant la revendication 1 ou 2, **caractérisés en ce qu'**ils se présentent sous la forme d'hydrogels.

4. Matériaux de support à base d'alcool polyvinylique suivant l'une des revendications 1 à 3, **caractérisés en ce qu'**ils se présentent sous la forme de particules en forme de perles ou de boules.

5. Matériaux de support à base d'alcool polyvinylique suivant la revendication 4, **caractérisés en ce que** les particules présentent une taille de 0,2 à 50 µm, de préférence de 0,5 à 5 µm.

6. Matériaux de support à base d'alcool polyvinylique suivant la revendication 1, **caractérisés en ce qu'**ils se présentent sous la forme d'un filtre ou d'une membrane.

7. Procédé de préparation d'un matériau de support magnétique à base d'alcool polyvinylique, modifié en surface, suivant l'une des revendications 1 à 6, **caractérisé en ce qu'**on fait réagir le matériau de support à base d'alcool polyvinylique avec un composé de silane organique
- de la formule générale (I) :
Xq-Si-(OR)_{4-q} (I)
dans laquelle
q est un nombre entier de 0 à 3,
R sont identiques ou différents et représentent de l'hydrogène, un radical alkyle, de préférence en C₁-C₆, particulièrement avantageusement en C₁-C₂, un radical aryle, de préférence un radical phényle,
et
X sont identiques ou différents et représentent de l'hydrogène, un radical alkyle, de préférence en C₁-C₂, un radical aryle, de préférence un radical phényle, ou un halogène, de préférence du chlore,
ou
- de la formule générale (II) :
(Y-R¹)_{q}-Si(OR)_{4-q} (II)
dans laquelle
R, q ont la signification donnée pour la formule générale (I),
R¹ représente un radical alkylène en C₁-C₆, de préférence un radical éthylène ou propylène,
Y représente un groupe amino, un groupe dialkylamino, de préférence un groupe diméthylamino ou diéthylamino, SH, un groupe époxy, un groupe vinyle, de préférence un groupe -CR²=CR³₂, où R² et respectivement R³ sont identiques ou différents et représentent de l'hydrogène, un radical alkyle, de préférence en C₁-C₂, un radical
aryle, de préférence un radical phényle, ou un radical d'acide acrylique,
ou
- avec un composé de silane polymère présentant l'unité répétitive de la formule générale (III) : 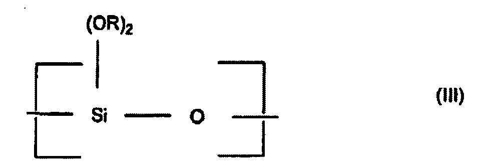 dans laquelle R, qui a la signification donnée dans la formule générale (I), est de préférence un radical méthyle.

8. Procédé suivant la revendication 7, **caractérisé en ce que** la réaction a lieu à des températures de 70 à 150°C, de préférence de 80 à 100°C.

9. Procédé suivant la revendication 7 ou 8, **caractérisé en ce que** le matériau de support à base d'alcool polyvinylique, de préférence les particules d'alcool polyvinylique, est (sont), pour la réaction, mis (es) en suspension dans un milieu aqueux, de préférence à un pH de 2 à 7.

10. Procédé suivant l'une des revendications 7 à 9, **caractérisé en ce que** le matériau de support à base d'alcool polyvinylique, de préférence les particules d'alcool polyvinylique, est (sont), pour la réaction, mis (es) en suspension dans un solvant organique, hydrophobe.

11. Procédé suivant l'une des revendications 7 à 10, **caractérisé en ce que** la réaction a lieu pendant une durée de 1 minute à 48 heures, de préférence avec mélange intime du matériau de support à base d'alcool polyvinylique et du composé de silane.

12. Procédé suivant l'une des revendications 7 à 11, **caractérisé en ce que** les groupes OH à la surface du matériau de support à base d'alcool polyvinylique sont mis à réagir avec 1,2 à 1,8 fois la quantité équivalente du composé de silane.

13. Procédé suivant l'une des revendications 7 à 12, **caractérisé en ce que**, après la réaction avec un composé de silane de la formule générale (I) à (III), éventuellement après isolement à partir du milieu réactionnel et purification, le matériau de support à base d'alcool polyvinylique est mis à réagir encore une fois avec un composé de silane de la formule générale (II).

14. Utilisation de matériaux de support à base d'alcool polyvinylique suivant les revendications 1 à 6 ou que l'on peut obtenir suivant les revendications 7 à 13, pour l'isolement et/ou la purification d'acides nucléiques issus d'échantillons biologiques, **caractérisée en ce qu'**on met en oeuvre les matériaux de support à base d'alcool polyvinylique pour l'immobilisation et l'isolement des acides nucléiques.

15. Utilisation suivant la revendication 14, **caractérisée en ce que** l'échantillon biologique est additionné d'une solution tampon appropriée pour l'extraction des acides nucléiques.

16. Utilisation suivant la revendication 15, **caractérisée en ce que** l'échantillon biologique est, avant l'extraction, additionné d'un agent, de préférence une RNAse ou un alcali, qui détruit l'acide ribonucléique (ARN).
